# EUROPEAN PATENT SPECIFICATION

(11) **EP 2 786 139 B1**
(45) Date of publication and mention of the grant of the patent: **24.02.2016**
(21) Application number: 12797965.6
(22) Date of filing: 03.12.2012
(51) Int. Cl.: G01N 33/487

(54) **HAND-HELD TEST METER WITH ANALYTICAL TEST STRIP EJECTION MECHANISM**
TRAGBARE MESSVORRICHTUNG MIT EINEM MECHANISMUS ZUR AUSGABE VON ANALYSETESTSTREIFEN
APPAREIL DE MESURE D'ESSAI PORTABLE À MÉCANISME D'ÉJECTION DE BANDELETTE D'ESSAI ANALYTIQUE

(30) Priority: 02.12.2011 US 201113310137
(43) Date of publication of application: 08.10.2014
(73) Proprietor: Lifescan Scotland Limited, Inverness IV2 3ED (GB)
(72) Inventor: NELSON, Jonny, Inverness IV2 5FW (GB); FAULKNER, Allan, Avoch IV9 8PT (GB); VALSECCHI, Luca, Milano 20126 (IT); BERETTA, Roberto, I-20063 Cernusco sul Naviglio (IT); VOLPE, Maurizio, I-10035 Mazzè (IT); SALA, Michele, I-20862 Arcore (IT); FOLEY, Nick, Edinburgh EH5 3RB (GB); CROSSLAND, Colin, Edinburgh EH9 3HW (GB); TRICKETT, Paul, Hamilton ML3 6BG (GB)
(74) Representative: Carpmaels & Ransford LLP
(86) International application number: PCT/GB2012/052987
(87) International publication number: WO 2013/079977

(56) References cited:
- EP-A1- 1 762 848
- EP-A1- 1 897 488
- WO-A2-2009/055643

## Description

### BACKGROUND OF THE INVENTION

### Field of the Invention

The present invention relates, in general, to medical devices and, in particular, to test meters and related methods.

### Description of Related Art

The determination (e.g., detection and/or concentration measurement) of an analyte in a fluid sample is of particular interest in the medical field. For example, it can be desirable to determine glucose, ketone bodies, cholesterol, lipoproteins, triglycerides, acetaminophen and/or HbA1c concentrations in a sample of a bodily fluid such as urine, blood, plasma or interstitial fluid. Such determinations can be achieved using a hand-held test meter in combination with analytical test strips (e.g., electrochemical-based analytical test strips).

During use, a single analytical test strip is typically inserted into a hand-held test meter. Following determination of an analyte in a bodily fluid sample applied to the analytical test strip, it is conventional for the analytical test strip to be manually removed from the hand-held test meter by a user and discarded. Conventional approaches to inserting and removing a test strip from a hand-held test meter are described in, for example, U.S. Patents 5,266,179; 5,366,609; and 5,738,244; and U.S. Patent Application Number 2009/0108013.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIGs. 1A and 1B are simplified top and cross-sectional side view block diagrams, respectively, of a hand-held test meter according to an embodiment of the present invention;
FIG. 2 is a simplified, exploded, perspective depiction of a housing, a test strip ejection mechanism and strip port connector as can be employed in hand-held test meters according to embodiments of the present invention;
FIG. 3 is a simplified perspective depiction of the test strip ejection mechanism and strip port connector of FIG. 2 with arrow A indicating the location and direction of analytical test strip ejection;
FIG. 4 is a simplified cross-sectional side view of the housing, test strip ejection mechanism and strip port connector of FIG. 2 depicting an analytical test strip operatively engaged with a test strip slider of the test strip ejection mechanism;
FIG. 5 is a simplified cross-sectional side view of the housing, test strip ejection mechanism and strip port connector of FIG. 2 depicting an analytical test strip in the process of being ejected from the test strip ejection mechanism; and
FIG. 6 is a flow diagram depicting stages in a method for ejecting an analytical test strip from a hand-held test meter according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein.

In general, hand-held test meters for use with an analytical test strip in the determination of an analyte (such as glucose) in a bodily fluid sample (e.g., a whole blood sample) according to embodiments of the present invention include a housing (with an outer surface) and an analytical test strip ejection mechanism. The analytical test strip ejection mechanism has an actuation button disposed in the outer surface of the housing, a motion amplification and rotation assembly operatively connected to the actuation button, and a test strip slider operatively connected to the motion amplification and rotation assembly. The actuation button is configured for movement by a user's digit in a first direction and the motion rotation and amplification assembly and test strip slider are configured to convert the movement in the first direction into an amplified (i.e., greater movement) of the test strip slider in a second direction with the second direction being rotated with respect to the first direction. In addition, the test strip slider is further configured for operative engagement with an analytical test strip inserted into the hand-held test meter in an engaged state such that movement of the test strip slider in the second direction from the engaged state to an ejected state ejects the analytical test strip from the hand-held test meter.

Such hand-held test meters are particularly beneficial in that a user is able to eject and, therefore, discard a used analytical test strip without directly contacting the analytical test strip. This reduces the risk of user exposure to blood-born pathogens or other contaminants that may be present on the surface of the used analytical test strip. Since the distance of movement of the actuation button is amplified (i.e., increased by, for example, a factor of two) by the motion amplification and rotation mechanism, movement of the actuation button a relatively small distance suffices to move the test strip slider a greater distance sufficient to eject the analytical test strip. This eases operation of the hand-held test meter for a user. In addition, since the ejection of the analytical test strip is mechanically controlled by a user's initiation of the actuation button, the user can beneficially determine the timing, force and speed of analytical test strip ejection.

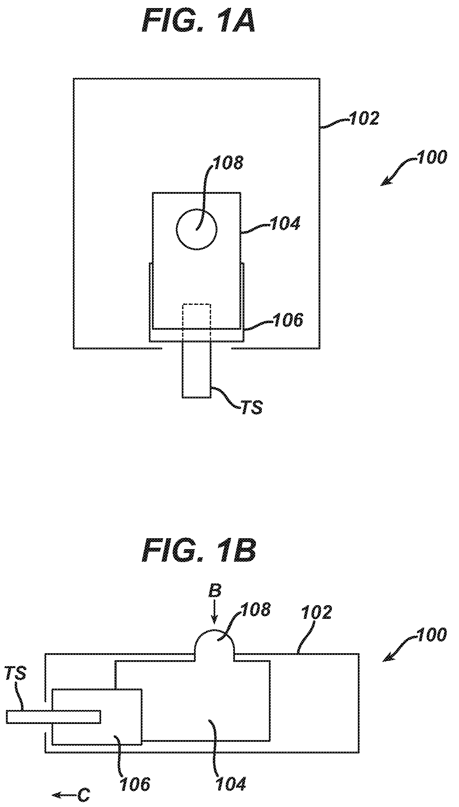

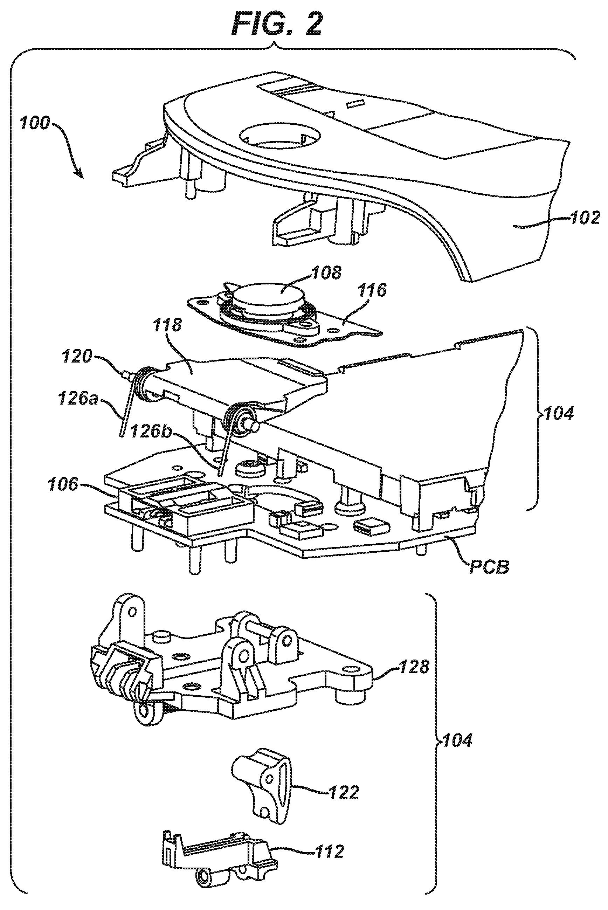

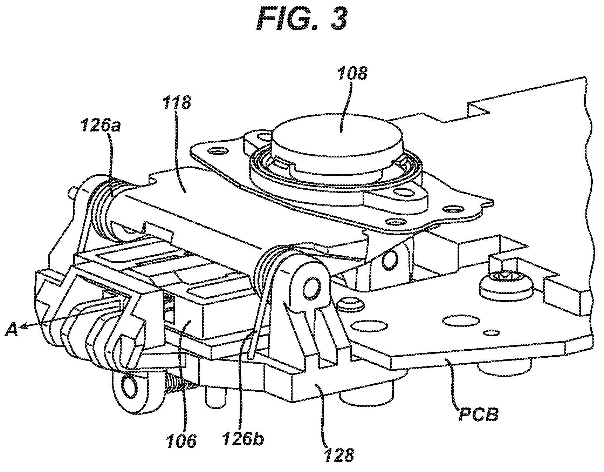

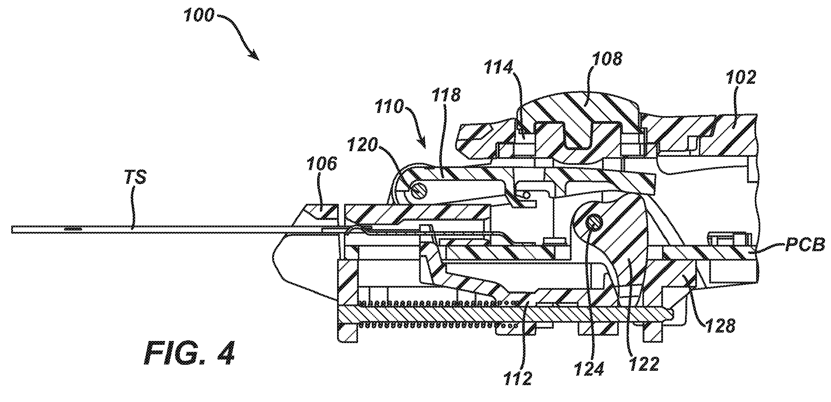

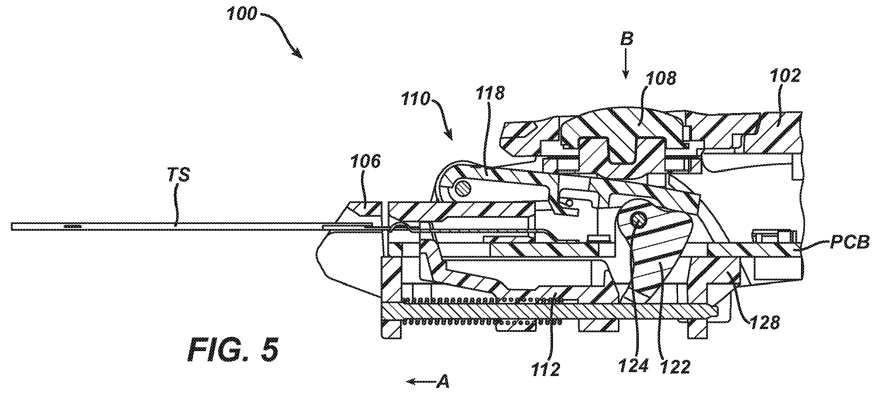

FIGs. 1A and 1B are simplified top and cross-sectional side view block diagrams, respectively, of a hand-held test meter 100 according to an embodiment of the present invention including a housing 102, a test strip ejection mechanism 104 and a strip port connector 106. Also depicted in FIGs. 1A, 1B, 4 and 5 is an analytical test strip (such as an electrochemical-based analytical test strip configured for the determination of glucose in a whole blood sample). FIG. 2 is a simplified, exploded, perspective depiction of a housing 102, a test strip ejection mechanism 104 and strip port connector 106 of hand-held test meter 100. FIG. 3 is a simplified perspective depiction of test strip ejection mechanism 104 and strip port connector 106 of FIG. 2. FIG. 4 is a simplified cross-sectional side view of housing 102, test strip ejection mechanism 104 and strip port connector 106 depicting an analytical test strip (TS) operatively engaged with a test strip slider of test strip ejection mechanism 104. FIG. 5 is a simplified cross-sectional side view of housing 102, test strip ejection mechanism 104 and strip port connector 106 depicting the analytical test strip (TS) in the process of being ejected from the hand-held test meter.

Referring to FIGs. 1A through 5, hand-held test meter 100 includes a housing 102 with an outer surface, a test strip ejection mechanism 104 and a strip port connector 106.

A test strip ejection mechanism 104 includes an actuation button 108 disposed in the outer surface of housing 102 (see FIGs. 1B, 4 and 5 in particular), a motion amplification and rotation assembly 110 operatively connected to actuation button 108, and a test strip slider 112 operatively connected to motion amplification and rotation assembly 110.

Actuation button 108 is configured for movement by a user's digit (i.e., a user's finger or thumb) in a first direction (see arrow B of FIGs. 1B and 5). Membrane 114 and button support plate 116 of test strip ejection mechanism 104 are configured to seal actuation button 108 against liquid ingress by the providing a liquid tight compression fit between rubber membrane 114, housing 102 and button support plate 116.

Motion amplification and rotation assembly 110 (numbered only generally in FIGs. 4 and 5 for clarity) includes a lever 118, a lever pin 120, a cam 122, a cam pin 124, and lever returning springs 126a and 126b. Test strip ejection mechanism 104 also includes an ejection mechanism frame 128.

Motion rotation and amplification assembly 110 and test strip slider 112 (e.g., a plastic slider) are configured to mechanically convert movement of actuation button 108 in the first direction (for example, the direction of arrow B in FIGs. 1B and 2) into greater movement of test strip slider 112 in a second direction (depicted by arrow C of FIG. 1A and arrow A of FIGs. 3 and 5). Moreover, motion rotation and amplification assembly 110 and test strip slider 112 are also configured such that the second direction is rotated with respect to the first direction. In hand-held test meter 100, the rotation is a 90 degree counter-clockwise rotation in the perspective of FIG. 1B. Moreover, the distance of movement of actuation button 108 in the first direction can be, for example, in the range of 1.6mm to 1.8mm while the amplified distance of movement of slider 112 in the second direction can be, for example, in the range of 3.0mm to 3.4mm.

In the embodiment of hand-held test meter 100, motion rotation and amplification assembly 110 includes a lever, a lever pin, a cam, a cam pin and returning springs. However, once apprised of the present disclosure, one skilled in the art could devise other equivalent mechanical configurations that serve the same purpose of converting movement of an actuation button in one direction into greater movement of a test strip slider in a second direction.

In the embodiment of FIGs. 1A through 5, lever 118 and cam 122 are configured to provide the aforementioned amplification and rotation in the following manner. As a user initiates activation of test strip ejection mechanism 104 by pressing on actuation button 108 with a force of, for example, approximately 3.7N, actuation button 108 mechanically acts on (i.e., applies a force to) lever 118 and moves lever 118 against the force of lever returning springs 126a and 126b. Lever 118 rotates about lever pin 120 (which is configured to provide support to lever 118 from ejection mechanism frame 128) as evidenced by a comparison of FIGs. 4 and 5. As lever 118 rotates, lever 118 mechanically acts on cam 122 such that cam 122 rotates about cam pin 124 (which is also configured to provide support to lever 118 from ejection mechanism frame 128) as also evidenced by a comparison of FIGs. 4 and 5. As cam 122 rotates, cam 122 acts on test strip slider 112 such that test strip slider 112 ejects analytical test strip (TS) from strip port connector 106 and hand-held test meter 100. In this manner, lever 118 and cam 122 of motion amplification and rotation assembly 110 serve to rotate the movement of actuation button 108 in a first direction into a greater movement of test strip slider 112 in a second direction. Such a greater movement can be, for example, greater by a factor of two (i.e., movement across a distance that is twice the distance of the movement of actuation button 108). This sequence is illustrated in FIGs. 4 and 5.

Test strip slider 112 further configured for operative engagement with an analytical test strip (TS) inserted into strip port connector 106 of hand-held test meter 100 in an engaged state such that movement of the test strip slider in the second direction from the engaged state to an ejected state ejects the analytical test strip from the hand-held test meter. Such an engaged state is depicted in FIG. 4 and such an ejected state is depicted in FIG. 5.

Test strip slider 112 moves along ejection mechanism frame 128 and encounters a hard stop against the ejection mechanism frame when in the ejected state. Upon release of actuation button 108 by a user, test strip slider 112 is returned to the engaged state by a helical spring (not shown) that acts between the test strip slider and the ejection mechanism frame. To optimize the force of this helical spring, lever 118 and, therefore, actuation button 108 are acted on by lever returning springs 126a and 126b.

Strip port connector 106 is configured to operatively receive an analytical test strip and, in the embodiment of FIGs. 1A-5, is connected to a printed circuit board (PCB) of hand-held test meter 100.

The components of hand-held test meter 100 described herein can be formed of any suitable materials known to one skilled in the art. For example, actuation button 108, lever 118, cam 122, ejection mechanism frame 128 and test strip slider 122 can be formed of a suitable plastic material and lever pin 120, cam pin 124, button support plate 116, and lever returning springs 126a and 126 be can be formed of a suitable stainless steel. In addition, membrane 114 can be formed of a suitable rubber material.

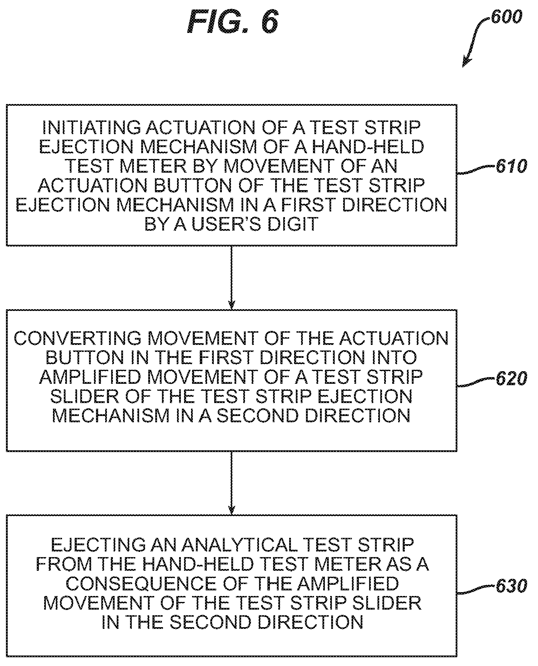

FIG. 6 is a flow diagram depicting stages in a method 600 for ejecting an analytical test strip from a hand-held test meter. Method 600 includes, at step 610 of FIG. 6, initiating actuation of a test strip ejection mechanism of a hand-held test meter in an engaged state by the movement of an actuation button of the test strip ejection mechanism in a first direction by a user's digit (i.e., finger or thumb). In the engaged state of step 610, an analytical test strip has been received within the hand-held test meter and is operatively engaged with a test strip slider of the test strip ejection mechanism.

At step 620, movement of the actuation button in the first direction is converted into amplified movement of the test strip slider in a second direction via action of the test strip ejection mechanism. Method 600 also includes ejecting the analytical test strip from the hand-held test meter as a consequence of the amplified movement of the test strip slider in the second direction to an ejected state (see step 630).

Once apprised of the present disclosure, one skilled in the art will recognize that method 600 can be readily modified to incorporate any of the techniques, benefits and characteristics of test strip ejection mechanisms and hand-held test meters according to embodiments of the present invention and described herein.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A hand-held test meter (100) for use with an analytical test strip in the determination of an analyte in a bodily fluid sample, the hand-held test meter comprising:
a housing (112) with an outer surface;
an analytical test strip ejection mechanism (104) including:
an actuation button (108) disposed in the outer surface of the housing;
a motion amplification and rotation assembly (110) operatively connected to the actuation button (108) and
a test strip slider (112) operatively connected to the motion amplification and rotation assembly (110),
wherein the actuation button is configured for movement by a user's digit in a first direction, and
wherein the motion rotation and amplification assembly and test strip slider are configured to convert movement of the actuation button in the first direction into an amplified movement of the test strip slider in a second direction, the second direction being rotated with respect to the first direction, and
wherein the test strip slider is further configured for operative engagement with an analytical test strip inserted into the hand-held test meter in an engaged state such that movement of the test strip slider in the second direction from the engaged state to an ejected state ejects the analytical test strip from the hand-held test meter.

2. The hand-held test meter of claim 1 wherein the actuation button is sealed against liquid ingress into the analytical test strip ejection mechanism.

3. The hand-held test meter of claim 1 wherein the second direction is rotated 90 degrees with respect to the first direction.

4. The hand-held test meter of claim 1 wherein the amplified motion of the test strip slider in the second direction is at least two times greater in distance than a distance of motion of the actuation button in the first direction.

5. The hand-held test meter of claim 1 wherein the motion amplification and rotation assembly includes:
a cam (122); and
a lever (118),
wherein movement of the actuation button in the first direction acts on the lever and the lever consequently acts on the cam to provide motion amplification and rotation.

6. The hand-held test meter of claim 5 wherein the cam and lever configured to amplify the first movement by a factor of at least two.

7. The hand-held test meter of claim 1 wherein the analytical test strip ejection mechanism further includes at least one torsion spring that acts upon the actuation button.

8. The hand-held test meter of claim 1 wherein the movement in the first direction is in the range of 1.6 mm to 1.8 mm and the movement in the second direction is in the range of 3.0 mm to 3.4 mm.

9. The hand-held test meter of claim 1 further including a strip port connector configured to receive the analytical test strip.

10. The hand-held test meter of claim 1 wherein the hand-held test meter is configured for the determination of glucose in a whole blood sample using an electrochemical-based analytical test strip.

11. A method for ejecting an analytical test strip from a hand-held test meter, the method comprising:
initiating actuation of a test strip ejection mechanism of a hand-held test meter in an engaged state by the movement of an actuation button of the test strip ejection mechanism in a first direction by a user's digit, wherein in the engaged state an analytical test strip has been received within the hand-held test meter and is operatively engaged with a test strip slider of the test strip ejection mechanism;
converting movement of the actuation button in the first direction into amplified movement of the test strip slider in a second direction via action of the test strip ejection mechanism; and
ejecting the analytical test strip from the hand-held test meter as a consequence of the amplified movement of the test strip slider in the second direction to an ejected state.

12. The method of claim 11 further comprising:
returning, following ejecting of the analytical test strip, the test strip slider to the engaged state.

13. The method of claim 11 wherein during the converting a cam and a lever of the test strip ejection mechanism are employed to convert movement in the first direction into amplified movement in the second direction.

14. The method of claim 11 wherein the second direction is rotated 90 degrees with respect to the first direction.

15. The method of claim 11 wherein the amplified movement of the test strip slider in the second direction is at least two times greater than the motion of the actuation button in the first direction.

16. The method of claim 11 wherein the movement in the first direction is in the range of 1.6 mm to 1.8 mm and the movement in the second direction is in the range of 3.0 mm to 3.4 mm.

17. The method of claim 11 further comprising:
determining, prior initiating actuation of the test strip ejection mechanism, an analyte in a bodily fluid sample applied to the analytical test strip.

18. The method of claim 17 wherein the analyte is glucose and bodily fluid sample is a whole blood sample.

19. The method of claim 17 wherein the analytical test strip is an electrochemical-based analytical test strip.

20. The method of claim 11 wherein the hand-held test meter is configured for the determination of glucose in a whole blood sample.

## Patentansprüche

1. Tragbares Testgerät (100) zum Gebrauch mit einem Analyseteststreifen zur Bestimmung eines Analyts in einer Körperflüssigkeitsprobe, wobei das tragbare Testgerät Folgendes umfasst:
ein Gehäuse (112) mit einer äußeren Oberfläche;
einen Analyseteststreifenauswurfmechanismus (104), der Folgendes umfasst:
eine Betätigungstaste (108), die an der äußeren Oberfläche des Gehäuses angeordnet ist;
eine Bewegungsverstärkungs- und Drehungsanordnung (110), die wirksam mit der Betätigungstaste (108) verbunden ist, und
eine Teststreifenschiebevorrichtung (112), die wirksam mit der Bewegungsverstärkungs- und Drehungsanordnung (110) verbunden ist,
wobei die Betätigungstaste zur Bewegung durch den Finger eines Anwenders in einer ersten Richtung konfiguriert ist, und
wobei die Bewegungsverstärkungs- und Drehungsanordnung und die Teststreifenschiebevorrichtung konfiguriert sind, die Bewegung der Betätigungstaste in der ersten Richtung in eine verstärkte Bewegung der Teststreifenschiebevorrichtung in eine zweite Richtung umzusetzen, wobei die zweite Richtung in Bezug auf die erste Richtung gedreht ist, und
wobei die Teststreifenschiebevorrichtung ferner konfiguriert ist zum wirksamen Einrasten eines Analyseteststreifens, der in das tragbare Testgerät eingeführt ist, in einem eingerasteten Zustand, so dass die Bewegung der Teststreifenschiebevorrichtung in der zweiten Richtung von dem eingerasteten Zustand zu einem ausgeworfenen Zustand den Analyseteststreifen aus dem tragbaren Testgerät auswirft.

2. Tragbares Testgerät nach Anspruch 1, wobei die Betätigungstaste gegen Eindringen von Flüssigkeit in den Analyseteststreifenauswurfmechanismus abgedichtet ist.

3. Tragbares Testgerät nach Anspruch 1, wobei die zweite Richtung um 90 Grad in Bezug auf die erste Richtung gedreht ist.

4. Tragbares Testgerät nach Anspruch 1, wobei die Strecke der verstärken Bewegung der Teststreifenschiebevorrichtung in der zweiten Richtung wenigstens zweimal größer ist als eine Bewegungsstrecke der Betätigungstaste in der ersten Richtung.

5. Tragbares Testgerät nach Anspruch 1, wobei die Bewegungsverstärkungs- und Drehungsanordnung Folgendes enthält:
einen Nocken (122); und
einen Hebel (118),
wobei die Bewegung der Betätigungstaste in der ersten Richtung auf den Hebel wirkt und der Hebel infolgedessen auf den Nocken wirkt, um Bewegungsverstärkung und Drehung bereitzustellen.

6. Tragbares Testgerät nach Anspruch 5, wobei der Nocken und der Hebel konfiguriert sind, die erste Bewegung um einen Faktor von wenigstens zwei zu verstärken.

7. Tragbares Testgerät nach Anspruch 1, wobei der Analyseteststreifenauswurfmechanismus ferner wenigstens eine Torsionsfeder, die auf die Betätigungstaste einwirkt, enthält.

8. Tragbares Testgerät nach Anspruch 1, wobei die Bewegung in der ersten Richtung im Bereich von 1,6 mm bis 1,8 mm ist und die Bewegung in der zweiten Richtung im Bereich von 3,0 mm bis 3,4 mm ist.

9. Tragbares Testgerät nach Anspruch 1, das ferner ein Streifenöffnungsverbindungselement enthält, das konfiguriert ist, den Analyseteststreifen aufzunehmen.

10. Tragbares Testgerät nach Anspruch 1, wobei das tragbare Testgerät zur Bestimmung von Glucose in einer Vollblutprobe unter Verwendung eines elektrochemiebasierten Analyseteststreifens konfiguriert ist.

11. Verfahren zum Ausstoßen eines Analyseteststreifens aus einem tragbaren Testgerät, wobei das Verfahren Folgendes umfasst:
einleitendes Betätigen eines Teststreifenauswurfmechanismus eines tragbaren Testgeräts in einem eingerasteten Zustand durch die Bewegung einer Betätigungstaste des Teststreifenauswurfmechanismus in eine erste Richtung durch den Finger eines Anwenders, wobei in dem eingerasteten Zustand ein Analyseteststreifen innerhalb des tragbaren Testgeräts aufgenommen worden ist und wirksam in eine Teststreifenschiebevorrichtung des Teststreifenauswurfmechanismus eingerastet ist;
Umsetzen der Bewegung der Betätigungstaste in der ersten Richtung in eine verstärkte Bewegung der Teststreifenschiebevorrichtung in eine zweiten Richtung über die Wirkung des Teststreifenauswurfmechanismus; und
Ausstoßen des Analyseteststreifens aus dem tragbaren Testgerät als eine Folge der verstärkten Bewegung der Teststreifenschiebevorrichtung in die zweite Richtung zu einem ausgeworfenen Zustand.

12. Verfahren nach Anspruch 11, das ferner Folgendes umfasst:
Zurückkehren nachfolgend auf das Ausstoßen des Analyseteststreifens der Teststreifenschiebevorrichtung in den eingerasteten Zustand.

13. Verfahren nach Anspruch 11, wobei während des Umsetzens ein Nocken und ein Hebel des Teststreifenauswurfmechanismus eingesetzt werden, um Bewegung in die erste Richtung in verstärkte Bewegung in die zweite Richtung umzusetzen.

14. Verfahren nach Anspruch 11, wobei die zweite Richtung um 90 Grad in Bezug auf die erste Richtung gedreht ist.

15. Verfahren nach Anspruch 11, wobei die verstärke Bewegung der Teststreifenschiebevorrichtung in die zweite Richtung wenigstens zweimal größer ist als die Bewegung der Betätigungstaste in die erste Richtung.

16. Verfahren nach Anspruch 11, wobei die Bewegung in die erste Richtung im Bereich von 1,6 mm bis 1,8 mm ist und die Bewegung in die zweite Richtung im Bereich von 3,0 mm bis 3,4 mm ist.

17. Verfahren nach Anspruch 11, das ferner Folgendes umfasst:
Bestimmen vor der einleitenden Betätigung des Teststreifenauswurfmechanismus eines Analyts in einer Körperflüssigkeitsprobe, die auf dem Analyseteststreifen aufgebracht ist.

18. Verfahren nach Anspruch 17, wobei der Analyt Glucose ist und die Körperflüssigkeitsprobe eine Vollblutprobe ist.

19. Verfahren nach Anspruch 17, wobei der Analyseteststreifen ein elektrochemiebasierter Analyseteststreifen ist.

20. Verfahren nach Anspruch 11, wobei das tragbare Testgerät zur Bestimmung von Glucose in einer Vollblutprobe konfiguriert ist.

## Revendications

1. Appareil de mesure de test portable (100) pour l'utilisation avec une bandelette de test analytique pour déterminer un analyte dans un échantillon de fluide corporel, l'appareil de mesure de test portable comprenant :
un boîtier (112) avec une surface extérieure ;
un mécanisme (104) d'éjection de la bandelette de test analytique comportant :
un bouton d'actionnement (108) disposé dans la surface extérieure du boîtier ;
un ensemble d'amplification de mouvement et de rotation (110) connecté fonctionnellement au bouton d'actionnement (108) et un coulisseau de bandelette de test (112) connecté fonctionnellement à l'ensemble d'amplification de mouvement et de rotation (110),
le bouton d'actionnement étant configuré en vue d'un déplacement par un doigt d'un utilisateur dans une première direction, et
l'ensemble d'amplification de mouvement et de rotation et le coulisseau de bandelette de test étant configurés pour convertir le mouvement du bouton d'actionnement dans la première direction en un mouvement amplifié du coulisseau de la bandelette de test dans une deuxième direction, la deuxième direction étant tournée par rapport à la première direction, et
le coulisseau de bandelette de test étant en outre configuré pour un engagement fonctionnel avec une bandelette de test analytique insérée dans l'appareil de mesure de test portable dans un état engagé, de telle sorte que le mouvement du coulisseau de bandelette de test dans la deuxième direction depuis l'état engagé dans un état éjecté éjecte la bandelette de test analytique hors de l'appareil de mesure de test portable.

2. Appareil de mesure de test portable selon la revendication 1, dans lequel le bouton d'actionnement est scellé contre l'entrée de liquide dans le mécanisme d'éjection de bandelette de test analytique.

3. Appareil de mesure de test portable selon la revendication 1, dans lequel la deuxième direction est tournée de 90 degrés par rapport à la première direction.

4. Appareil de mesure de test portable selon la revendication 1, dans lequel le mouvement amplifié du coulisseau de bandelette de test dans la deuxième direction est au moins deux fois plus grand, en termes de distance, qu'une distance de déplacement du bouton d'actionnement dans la première direction.

5. Appareil de mesure de test portable selon la revendication 1, dans lequel l'ensemble d'amplification de mouvement et de rotation comporte :
une came (122) ; et
un levier (118),
le mouvement du bouton d'actionnement dans la première direction agissant sur le levier et le levier agissant par conséquent sur la came pour assurer l'amplification du mouvement et la rotation.

6. Appareil de mesure de test portable selon la revendication 5, dans lequel la came et le levier sont configurés pour amplifier le premier mouvement d'un facteur d'au moins deux.

7. Appareil de mesure de test portable selon la revendication 1, dans lequel le mécanisme d'éjection de la bandelette de test analytique comporte en outre au moins un ressort de torsion qui agit sur le bouton d'actionnement.

8. Appareil de mesure de test portable selon la revendication 1, dans lequel le mouvement dans la première direction est de l'ordre de 1,6 mm à 1,8 mm et le mouvement dans la deuxième direction est de l'ordre de 3,0 mm à 3,4 mm.

9. Appareil de mesure de test portable selon la revendication 1, comportant en outre un connecteur d'orifice de bandelette configuré pour recevoir la bandelette de test analytique.

10. Appareil de mesure de test portable selon la revendication 1, dans lequel l'appareil de mesure de test portable est configuré pour la détermination de glucose dans un échantillon de sang total en utilisant une bandelette de test analytique à base électrochimique.

11. Procédé d'éjection d'une bandelette de test analytique d'un appareil de mesure de test portable, le procédé comprenant :
l'amorçage de l'actionnement d'un mécanisme d'éjection de bandelette de test d'un appareil de mesure de test portable dans un état engagé par le mouvement d'un bouton d'actionnement du mécanisme d'éjection de bandelette de test dans une première direction par un doigt d'un utilisateur, une bandelette de test analytique, dans l'état engagé, ayant été reçue à l'intérieur de l'appareil de mesure de test portable et étant engagée fonctionnellement avec un coulisseau de bandelette de test du mécanisme d'éjection de bandelette de test ;
la conversion du mouvement du bouton d'actionnement dans la première direction en un mouvement amplifié du coulisseau de bandelette de test dans une deuxième direction par l'action du mécanisme d'éjection de bandelette de test ; et
l'éjection de la bandelette de test analytique de l'appareil de mesure de test portable en conséquence du mouvement amplifié du coulisseau de bandelette de test dans la deuxième direction jusque dans un état éjecté.

12. Procédé selon la revendication 11, comprenant en outre :
le retour, suite à l'éjection de la bandelette de test analytique, du coulisseau de bandelette de test dans l'état engagé.

13. Procédé selon la revendication 11, dans lequel, au cours de la conversion, une came et un levier du mécanisme d'éjection de bandelette de test sont utilisés pour convertir le mouvement dans la première direction en un mouvement amplifié dans la deuxième direction.

14. Procédé selon la revendication 11, dans lequel la deuxième direction est tournée de 90 degrés par rapport à la première direction.

15. Procédé selon la revendication 11, dans lequel le mouvement amplifié du coulisseau de bandelette de test dans la deuxième direction est au moins deux fois plus important que le mouvement du bouton d'actionnement dans la première direction.

16. Procédé selon la revendication 11, dans lequel le mouvement dans la première direction est de l'ordre de 1,6 mm à 1,8 mm et le mouvement dans la deuxième direction est de l'ordre de 3, 0 mm à 3, 4 mm.

17. Procédé selon la revendication 11, comprenant en outre :
la détermination, avant d'amorcer l'actionnement du mécanisme d'éjection de bandelette de test, d'un analyte dans un échantillon de fluide corporel appliqué sur la bandelette de test analytique.

18. Procédé selon la revendication 17, dans lequel l'analyte est du glucose et l'échantillon de fluide corporel est un échantillon de sang total.

19. Procédé selon la revendication 17, dans lequel la bandelette de test analytique est une bandelette de test analytique à base électrochimique.

20. Procédé selon la revendication 11, dans lequel l'appareil de mesure de test portable est configuré pour la détermination de glucose dans un échantillon de sang total.
